(19) Europäisches Patentamt
European Patent Office
Office européen des brevets

(11) **EP 3 138 546 B1**

(12) **EUROPEAN PATENT SPECIFICATION**

(45) Date of publication and mention
of the grant of the patent:
**01.08.2018 Bulletin 2018/31**

(21) Application number: **15786777.1**

(22) Date of filing: **03.04.2015**

(51) Int Cl.:
**A61F 13/496** (2006.01)    **A61F 13/49** (2006.01)

(86) International application number:
**PCT/JP2015/060623**

(87) International publication number:
**WO 2015/166766 (05.11.2015 Gazette 2015/44)**

(54) **DISPOSABLE DIAPER AND METHOD FOR MANUFACTURING DISPOSABLE DIAPER**

EINWEGWINDEL UND VERFAHREN ZUR HERSTELLUNG EINER EINWEGWINDEL

COUCHE JETABLE ET PROCÉDÉ DE PRODUCTION DE COUCHE JETABLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.05.2014 JP 2014095526**

(43) Date of publication of application:
**08.03.2017 Bulletin 2017/10**

(73) Proprietor: **Unicharm Corporation**
**Ehime 799-0111 (JP)**

(72) Inventors:
• **OKUDA, Jun**
**Kanonji-shi**
**Kagawa 769-1602 (JP)**
• **MITSUNO, Satoshi**
**Kanonji-shi**
**Kagawa 769-1602 (JP)**

(74) Representative: **Peter, Julian**
**Staeger & Sperling**
**Partnerschaftsgesellschaft mbB**
**Sonnenstrasse 19**
**80331 München (DE)**

(56) References cited:
WO-A1-2013/054923    JP-A- 2007 527 777
JP-A- 2012 095 936    JP-A- 2014 068 907
JP-A- 2014 198 143    US-A1- 2011 251 576

EP 3 138 546 B1

**Description**

**Technical Field**

The present invention relates to a disposable diaper and method for manufacturing a disposable diaper.

**Background Art**

A pants-type disposable diaper has been disclosed which is a disposable diaper provided with a compound stretchable member in the waistband section, the compound stretchable member being constructed with the outer side sheet and the inner side sheet mutually joined by intermittent joining sections in the expanding/shrinking direction of the compound stretchable member and in the direction orthogonal thereto, wherein elastic members are disposed so as not to pass through the joining sections and are not anchored to the outer side sheet and inner side sheet at the sections other than both ends of the elastic member, and the outer side sheet and the inner side sheet each form a plurality of folds continuously extending across a plurality of elastic members (see PTL 1, for example).

**Citation List**

**Patent literature**

PTL 1: Japanese Unexamined Patent Publication No. 2008-173286 Further prior art in this technical field is disclosed in documents US 2011/251576 A1, WO 2013/054923 A1 and EP 2 767 267 A

**Summary of Invention**

**Technical Problem**

It has been considered, however, to reduce the heights of the wrinkles (folds) of the compound stretchable member, i.e. the thickness during contraction, in order to achieve an improved fitting property of the waistband section in such a disposable diaper. Yet, this results in loss of the grip feel when the waistband section is gripped in order to lift the disposable diaper, potentially making it difficult to lift the disposable diaper.
Thus, for the compound stretchable member of the invention disclosed in PTL 1, it has been considered to widen the pitch of the wrinkles in order to increase the thickness during contraction. When the pitch of the wrinkles is widened, however, the joined surface area between the sheets joined together by the intermittent joining sections is reduced, and the joining strength is lower. As a result, when tension is applied to the compound stretchable member during use, the sheets separate from each other and the compound stretchable member can potentially break apart.
It is therefore an object of the present invention to provide a disposable diaper that has an improved fitting property of the waistband section of the disposable diaper and can be easily lifted when worn, as well as a method for producing such a disposable diaper.

**Solution to Problem**

In order to achieve the object stated above, the invention provides:

a disposable diaper comprising a waistband portion, situated around the waist of the wearer and a crotch portion situated at the crotch of the wearer, and extending from the stomach side portion of the waistband portion to the back side portion of the waistband portion,
a compound stretchable member being provided in the waistband portion,
the compound stretchable member comprising a plurality of elastic members extending in the waist direction and an inner side nonwoven fabric sheet portion and outer side nonwoven fabric sheet portion that are overlaid so as to be mutually joined through the elastic members,
wherein the inner side nonwoven fabric sheet portion and the outer side nonwoven fabric sheet portion each comprise a plurality of concavoconvex regions at least partially comprising protrusions and recesses that alternately repeat along the waist direction and extend in the direction orthogonal to the waist direction, and
at least one non-concavoconvex region mutually separating the concavoconvex regions in the direction orthogonal to the waist direction,
the waistband portion including a first region, and a second region provided either continuously with or separate from the first region in the direction orthogonal to the waist direction,

the protrusions and recesses of the concavoconvex regions being provided at a first concavoconvexity pitch with the compound stretchable member disposed in the first region, and at a second concavoconvexity pitch that is different from the first concavoconvexity pitch, in the compound stretchable member disposed in the second region, along the waist direction.

In order to achieve the object stated above the invention also provides a method for producing a disposable diaper comprising a waistband portion, situated around the waist of the wearer and a crotch portion situated at the crotch of the wearer, and extending from the stomach side portion of the waistband portion to the back side portion of the waistband portion, the method for producing a disposable diaper including at least:

a step of producing a compound stretchable member comprising an inner side nonwoven fabric sheet portion and an outer side nonwoven fabric sheet portion which are mutually overlaid, and a plurality of elastic members extending in a first direction between the inner side nonwoven fabric sheet portion and the outer side nonwoven fabric sheet portion, and
a step of forming the disposable diaper using the compound stretchable member at the waistband portion,
wherein the step of producing the compound stretchable member includes a shaping step, an overlaying step and a joining step,
in the shaping step, a plurality of concavoconvex regions at least partially comprising protrusions and recesses that alternately repeat along the first direction and extend in the second direction orthogonal to the first direction, and at least one non-concavoconvex region mutually separating the concavoconvex regions in the second direction, are formed in the inner side nonwoven fabric sheet portion and the outer side nonwoven fabric sheet portion, and the inner side nonwoven fabric sheet portion and the outer side nonwoven fabric sheet portion are shaped so that the compound stretchable member includes, in the concavoconvex regions, a first region with the protrusions and recesses provided at a first concavoconvexity pitch along the first direction, and in the concavoconvex region, a second region with the protrusions and recesses provided at a second concavoconvexity pitch which is different from the first concavoconvexity pitch, along the first direction, and so that the first region is continuous with or separate from the second region in the second direction,
in the overlaying step, the inner side nonwoven fabric sheet portion and the outer side nonwoven fabric sheet portion are overlaid,
in the joining step, the inner side nonwoven fabric sheet portion and the outer side nonwoven fabric sheet portion are joined together via the elastic members, and
in the step of forming the disposable diaper, the waistband portion is formed of the compound stretchable member by joining the compound stretchable member with the lengthwise ends of the absorbing element, so that the lengthwise direction of the absorbing element provided along the lengthwise direction of the crotch portion and the second direction of the compound stretchable member are flush.

**Advantageous Effects of Invention**

The disposable diaper of the invention includes first and second regions including concavoconvex regions in which protrusions and recesses are formed at mutually differing concavoconvexity pitches. This forms regions with an improved fitting property for the disposable diaper, and regions that are easy to grip and lift. As a result, it is possible to provide a disposable diaper which has an improved fitting property at the waistband portion of the disposable diaper, while also being easily lifted when worn.

**Brief Description of Drawings**

Fig. 1 is a schematic perspective view of a disposable diaper according to an embodiment of the invention.
Fig. 2 is a plan view schematically showing the spread-out state of the disposable diaper of Fig. 1. Shown is the skin side when worn.
Fig. 3(A) is a partial magnified view of the stomach side portion of the waistband portion of the disposable diaper of Fig. 3, and (B) is a cross-sectional view of the partial magnified view.
Fig. 4A is a magnified perspective view of the cut portion of section IVA of Fig. 3.
Fig. 4B is an exploded perspective view of Fig. 4A.
Fig. 5 is a magnified view of the periphery of the cross-section of the elastic member of Fig. 4A.
Fig. 6 is a schematic diagram of an apparatus for production of a compound stretchable member.
Fig. 7 is a perspective view of a cut-tooth gear roll and continuous gear roll in a shaping apparatus in the apparatus for production of a compound stretchable member.

Fig. 8 is a front view of the cut-tooth gear roll and continuous gear roll of Fig. 7.

Fig. 9A is a partial end view of small-pitch discontinuous teeth in the cut-tooth gear roll and small-pitch continuous teeth of the continuous gear roll of Fig. 7.

Fig. 9B is a partial end view of large-pitch discontinuous teeth in the cut-tooth gear roll and large-pitch continuous teeth of the continuous gear roll of Fig. 7.

Fig. 10A is a magnified cross-sectional view schematically showing the meshing section between a cut-tooth gear roll and a continuous gear roll, and a nonwoven fabric sheet deformed between them, at a cut-tooth portion of the cut-tooth gear roll, the view being linearly expanded in the peripheral direction of the cut-tooth gear roll and continuous gear roll.

Fig. 10B is a magnified cross-sectional view schematically showing the meshing section between a cut-tooth gear roll and a continuous gear roll, and a nonwoven fabric sheet deformed between them, at a discontinuous tooth portion of the cut-tooth gear roll, the view being linearly expanded in the peripheral direction of the cut-tooth gear roll and continuous gear roll.

Fig. 11 is a graph showing the height and length of a cross-section curve element, using a cross-section curve example.

Fig. 12 is a diagram illustrating the procedure for preparing a test strip.

## Description of Embodiments

The present invention will now be described in greater detail with reference to the accompanying drawings. It is to be understood that the drawings may not be drawn to the same size, scale or form for the actual constituent elements, in order to aid in understanding of the invention and to simplify the explanation of the drawings.

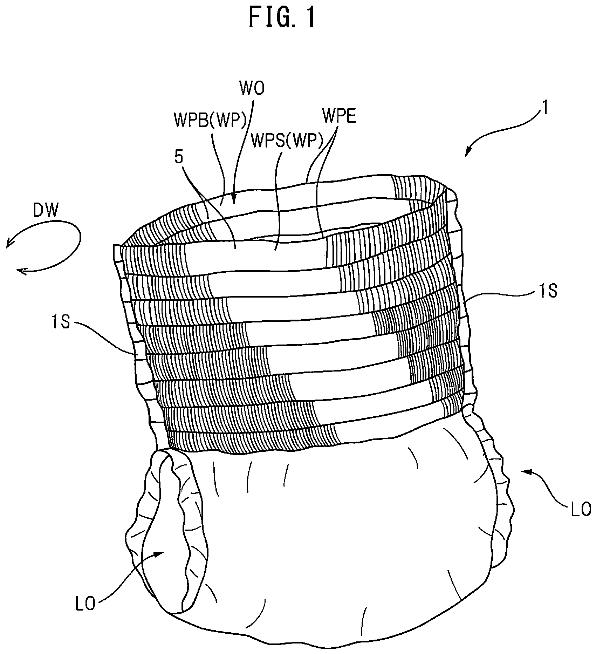

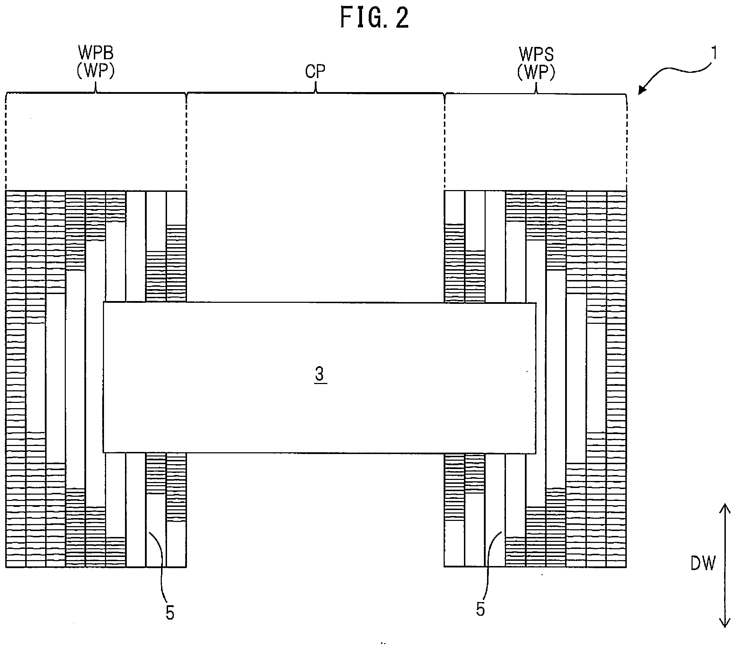

A disposable diaper 1 according to an embodiment of the invention will now be described with reference to Figs. 1 to 5. The disposable diaper 1 of this embodiment is an underwear-type disposable diaper. It is to be understood that in the drawings, some or all of the wrinkles formed in the disposable diaper 1 may not be shown in the drawings, for easier visualization of the drawings. The disposable diaper 1 shown in Fig. 2 is shown in a state spread out in a flat manner with the side section 1S of the disposable diaper 1 cut from the waistband opening WO to the leg opening LO, so that the wrinkles are stretched without creating excessive tension on the members composing the disposable diaper 1. In these spread-out views, the term "waist direction DW" is used as the definition of the direction, and the waist direction DW in these spread-out views is essentially orthogonal to the direction in which the crotch portion CP extends. Also, the "direction orthogonal to the waist direction DW" is the direction along the direction in which the compound stretchable member 5 extends, of the direction orthogonal to the waist direction DW described above.

The disposable diaper 1 of this embodiment comprises a waistband portion WP that covers the waist of the wearer over the entire waist direction DW when worn, and a crotch portion CP that covers the crotch of the wearer.

As shown in Fig. 1, the waistband portion WP includes a waistband portion edge WPE forming a waistband opening WO, located above the disposable diaper 1 when worn. The waistband portion WP includes a stomach side portion WPS located on the stomach side of the wearer when worn, and a back side portion WPB located on the back side of the wearer when worn. The stomach side portion WPS and back side portion WPB are joined by sonic sealing or heat sealing, for example, at each side section 1S of the disposable diaper 1. A compound stretchable member 5, described in detail below, is provided in the waistband portion WP.

As shown in Fig. 2, the crotch portion CP extends in the direction orthogonal to the waist direction DW, from the stomach side portion WPS to the back side portion WPB of the waistband portion WP. The crotch portion CP has a smaller dimension in the waist direction DW than in the waistband portion WP, whereby leg openings LO are formed in the disposable diaper 1. With this construction, as shown in Fig. 1, the waistband portion WP is located between the waistband opening WO and the leg openings LO, and the crotch portion CP forms the waistband portion WP and the leg openings LO.

The disposable diaper 1 of this embodiment comprises at least an absorbing element 3 forming the crotch portion CP, extending in the direction orthogonal to the waist direction DW from the stomach side portion WPS of the waistband portion WP to the back side portion WPB of the waistband portion WP. The absorbing element 3 has a liquid-impermeable back sheet located on the back surface on the side opposite the liquid-permeable top sheet which is located on the front surface of the wearer side when worn, and a liquid-absorbing absorbent body lying between the top sheet and the back sheet.

The top sheet of the absorbing element 3 is provided on the skin-contact side that contacts the skin of the wearer when it is worn. The top sheet is formed by a liquid-permeable sheet such as a hydrophilic nonwoven fabric.

The back sheet of the absorbing element 3 is provided on the opposite side of the top sheet. The back sheet may be formed from a leak-resistant (liquid-impermeable) plastic film or the like.

The absorbent body of the absorbing element 3 absorbs body fluids such as urine or feces of the wearer, and it is formed of an absorptive core of ground pulp, a super-absorbent polymer or the like, and an absorbent sheet such as a tissue that covers the absorptive core.

The compound stretchable member 5 provided on the waistband portion WP of the disposable diaper 1 comprises an outer side nonwoven fabric sheet portion 6L located on the outer side when worn, an inner side nonwoven fabric sheet portion 6U located on the side of the wearer's skin when worn, and a plurality of filamentous elastic members 7 extending in the waist direction DW between the nonwoven fabric sheet portions 6U, 6L. The inner side nonwoven fabric sheet portion 6U and the outer side nonwoven fabric sheet portion 6L are mutually overlaid, and joined together via the elastic members 7.

For this embodiment, an SMS nonwoven fabric with a basis weight of 15 g/m$^2$ is used as the inner side nonwoven fabric sheet portion 6U, and a spunbond nonwoven fabric with a basis weight of 17 g/m$^2$ is used as the outer side nonwoven fabric sheet portion 6L. However, the invention is in no way limited to this. A spunbond nonwoven fabric, SMS nonwoven fabric, air-through nonwoven fabric or the like may be used for each of the nonwoven fabric sheet portions 6U, 6L.

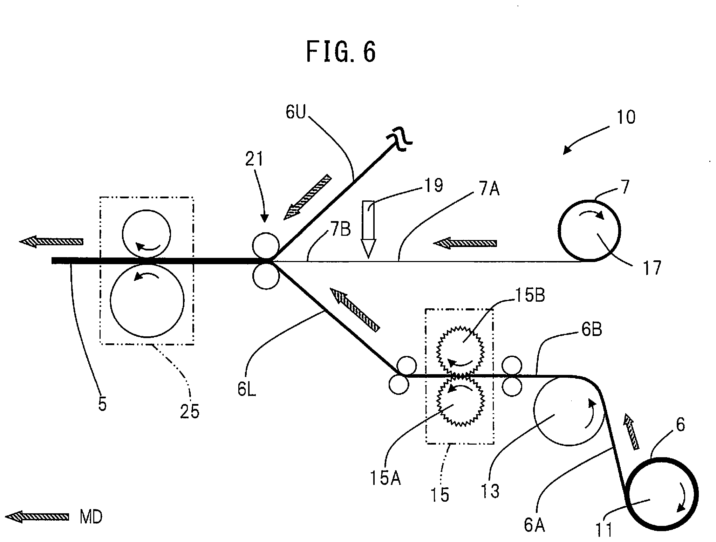

However, it is preferred to use for the nonwoven fabric sheet 6 a long filament nonwoven fabric formed by direct spinning without cutting the filaments, such as a spunbond nonwoven fabric, SMS nonwoven fabric, or the like. This is from the viewpoint of minimizing reduction in strength of the nonwoven fabric sheet 6 (Fig. 6) necessary for the shaping treatment described below, and because the ductility is high, while the nonwoven fabric sheet itself 6 will be thinner than using a staple fiber nonwoven fabric, and it will be possible to produce a fabric with high planarity (flatness).

Moreover, in this embodiment, the elastic members 7 used are multiple elastic yarn filaments made of urethane spandex, with sizes of 470 dtex and 780 dtex. However, the invention is in no way limited to this. For the elastic members 7 there are used several elastic yarn filaments of about 300 to 1200 dtex as the size, and preferably elastic yarn with the same size or with different sizes. This is because if the size is 300 dtex or smaller, the number of elastic yarn filaments used per unit width will increase, tending to require larger production equipment, and if it is 1200 dtex or greater, the spacing between adjacent elastic members will increase, potentially resulting in nonuniform meshing between the nonwoven fabric sheet portions 6U, 6L. Moreover, the material used for the elastic members 7 may be synthetic rubber such as styrene-butadiene, butadiene, isoprene or neoprene, natural rubber, EVA, SIS, SEBS, SEPS, expandable and shrinkable polyolefin, polyurethane, or the like.

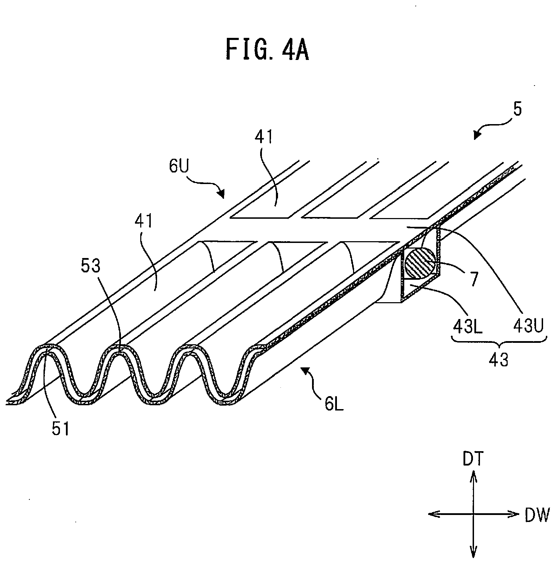

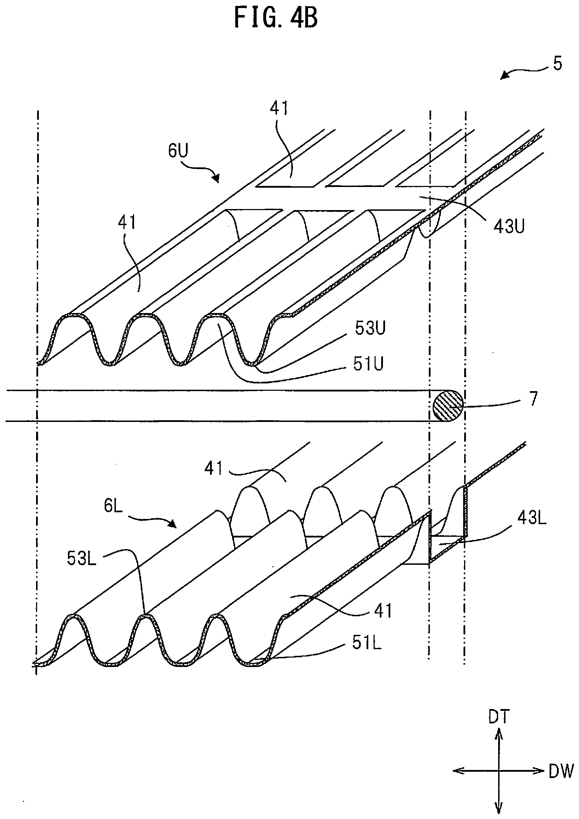

As shown in Figs. 4A and 4B, the inner side nonwoven fabric sheet portion 6U and the outer side nonwoven fabric sheet portion 6L each comprise a plurality of concavoconvex regions 41 comprising recesses 51 and protrusions 53 that alternately repeat along the waist direction DW, while extending in the direction orthogonal to the waist direction DW, and non-concavoconvex regions 43 mutually separating the concavoconvex regions 41 in the direction orthogonal to the waist direction DW. The inner side nonwoven fabric sheet portion 6U and the outer side nonwoven fabric sheet portion 6L need only comprise at least one non-concavoconvex region 43 each. Also, the concavoconvex regions 41 need only be provided at least partially with the recesses 51 and protrusions 53.

Also, in the compound stretchable member 5 of the disposable diaper 1 of this embodiment, as shown in Fig. 4A, the inner side nonwoven fabric sheet portion 6U and outer side nonwoven fabric sheet portion 6L are overlaid in such a manner that the concavoconvex regions 41 of the inner side nonwoven fabric sheet portion 6U and the concavoconvex regions 41 of the outer side nonwoven fabric sheet portion 6L are mutually overlaid and aligned, and the non-concavoconvex regions 43 of the inner side nonwoven fabric sheet portion 6U and the non-concavoconvex regions 43 of the outer side nonwoven fabric sheet portion 6L are mutually overlaid and aligned. Here, "concavoconvex regions 41 (or non-concavoconvex regions 43) are mutually aligned" means that the concavoconvex regions 41 of the inner side nonwoven fabric sheet portion 6U and the concavoconvex regions 41 of the outer side nonwoven fabric sheet portion 6L (the non-concavoconvex regions 43 of the inner side nonwoven fabric sheet portion 6U and the non-concavoconvex regions 43 of the outer side nonwoven fabric sheet portion 6L) extend parallel to each other.

Moreover, for this embodiment, in the concavoconvex regions 41, the protrusions 53U of the inner side nonwoven fabric sheet portion 6U enter into the recesses 51L of the outer side nonwoven fabric sheet portion 6L, and the protrusions 53L of the outer side nonwoven fabric sheet portion 6L enter into the recesses 51U of the inner side nonwoven fabric sheet portion 6U. Thus, the concavoconvex regions 41 of the inner side nonwoven fabric sheet portion 6U and the outer side nonwoven fabric sheet portion 6L are mutually adjacent in the thickness direction DT of the compound stretchable member 5. In contrast, the non-concavoconvex regions 43L, 43U formed in the inner side nonwoven fabric sheet portion 6U and the outer side nonwoven fabric sheet portion 6L are mutually separate in the thickness direction DT of the compound stretchable member 5.

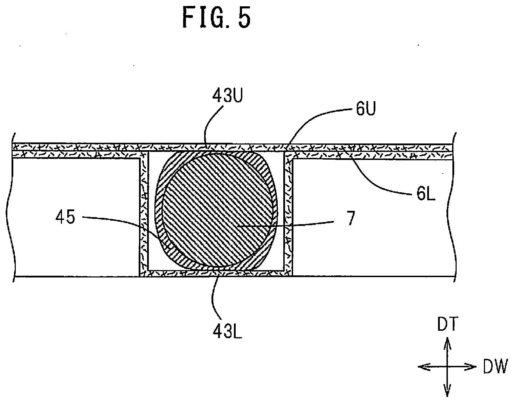

Also, as shown in Fig. 5, the two nonwoven fabric sheet portions 6U, 6L are joined together at bonding sections 45 by an adhesive via the elastic members 7 in the non-concavoconvex regions 43U, 43L. For this embodiment, the bonding sections 45 are dispersed so as to cover the entire peripheries of the elastic members 7.

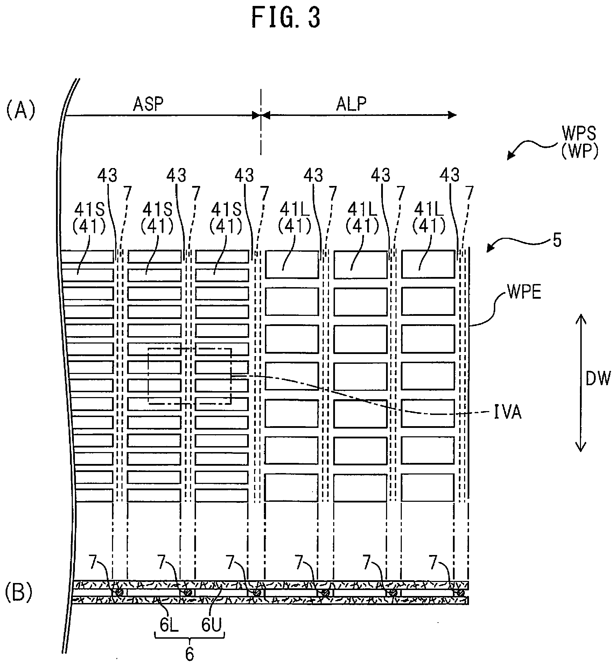

Incidentally, as shown in Fig. 3, the compound stretchable member 5 of the waistband portion WP of the disposable diaper 1 of this embodiment includes a large pitch area ALP located on the waistband opening WO side, and a small pitch area ASP located more toward the leg opening LO side than the large pitch area ALP. The concavoconvexity pitch of the concavoconvex regions 41S located in the small pitch area ASP (the distance between in the waist direction DW between the recesses 51 or protrusions 53 that are mutually adjacent in the waist direction DW) differs from the concavoconvexity pitch of the concavoconvex regions 41L located in the large pitch area ALP. For this embodiment, the concavoconvexity pitch of the concavoconvex regions 41L located in the large pitch area ALP is longer than the concavoconvexity pitch of the concavoconvex regions 41S located in the small pitch area ASP. Each of the concavoconvex regions 41S located in the small pitch area ASP are formed with approximately the same concavoconvexity pitch, and similarly, each of the concavoconvex regions 41L located in the large pitch area ALP are formed with approximately the same concavoconvexity pitch.

Throughout the present specification, one portion of the waistband portion WP including concavoconvex regions 41 formed at approximately a constant concavoconvexity pitch is considered to be a single region. With this, A plurality of regions having concavoconvex regions 41 formed with mutually different concavoconvexity pitches are provided in the waistband portion WP. The scope of the invention also includes a disposable diaper 1 including a waistband portion WP in which two or more regions are provided either continuously or separately in the direction orthogonal to the waist direction DW. For this embodiment, as shown in Fig. 3, the large pitch area ALP and the small pitch area ASP are provided continuously in the direction orthogonal to the waist direction DW. Between the large pitch area ALP and the small pitch area ASP there may be provided regions in which concavoconvex regions 41 are provided having a different concavoconvexity pitch than the large pitch area ALP and small pitch area ASP, or a different region such as a region where absolutely no recesses 51 and protrusions 53 are provided. Separate regions may also be provided further to the leg opening LO sides than the small pitch area ASP. In addition, separate regions may also be provided further to the waistband opening WO side than the large pitch area ALP.

In the compound stretchable member 5 of the disposable diaper 1 of this embodiment, the small pitch area ASP is provided at a location that overlaps the location where the absorbing element 3 is present, and the large pitch area ALP is provided at a location that does not overlap the location where the absorbing element 3 is present.

The description above concerns mainly the stomach side portion WPS of the waistband portion WP, as it pertains to the waistband portion WP. However, in the disposable diaper 1 of this embodiment, though not shown here, the back side portion WPB of the waistband portion WP has a similar construction to the stomach side portion WPS of the waistband portion WP. Thus, in the waistband portion WP of the disposable diaper 1 of this embodiment, the large pitch area ALP and small pitch area ASP of the compound stretchable member 5 are provided across the entire circumference in the waist direction DW of the waistband portion WP.

An example of a method for producing the compound stretchable member 5 to form the waistband portion WP for this embodiment will now be described with reference to Figs. 6 to 9B.

Fig. 6 is a schematic diagram of an apparatus 10 for production of a compound stretchable member 5. First, as a summary explanation of an example of the manufacturing method, in the apparatus 10, concavoconvex regions 41 and non-concavoconvex regions 43 are formed in the nonwoven fabric sheet 6 by a shaping apparatus 15, and formation of the concavoconvex regions 41 and non-concavoconvex regions 43 results in formation of an upper nonwoven fabric sheet 6U and lower nonwoven fabric sheet 6L, respectively. Next, at a merging zone 21, the upper nonwoven fabric sheet 6U and lower nonwoven fabric sheet 6L are overlaid. During this time, the elastic members 7 on which the adhesive has been applied at an adhesive applicator 19 are positioned between the non-concavoconvex regions 43 formed on the upper nonwoven fabric sheet 6U and lower nonwoven fabric sheet 6L. Finally, pressure is applied by an attachment press 25 onto the upper nonwoven fabric sheet 6U, lower nonwoven fabric sheet 6L and elastic members 7 in the thickness direction, thereby joining the upper nonwoven fabric sheet 6U and lower nonwoven fabric sheet 6L via the elastic members 7 and producing a compound stretchable member 5.

The nonwoven fabric sheet 6 composing the compound stretchable member 5 is kept wound on a sheet feeder 11, and it is first wound off from the sheet feeder 11 in the material machine direction MD and conveyed to a preheating roll 13. The preheating roll 13 preheats the wound out nonwoven fabric sheet 6A to render it more deformable, and for this example it is set to 50 to 130°C. The preheating temperature is determined according to the type and material of the nonwoven fabric sheet. According to a different production method, the apparatus 10 does not include a preheating roll 13, and conveying is directly to the shaping apparatus 15.

The preheated nonwoven fabric sheet 6B is then conveyed to the shaping apparatus 15. The shaping apparatus 15 is composed of a cut-tooth gear roll 15A and a continuous gear roll 15B, and for this example, the temperature is set to 50 to 130°C to facilitate shaping as with the preheating roll 13.

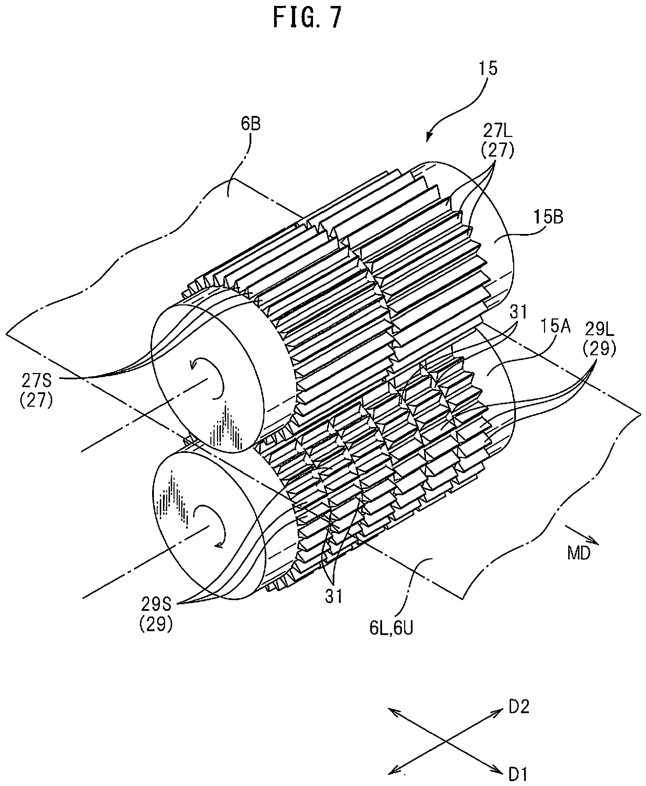

Fig. 7 is a perspective view of a cut-tooth gear roll 15A and a continuous gear roll 15B in a shaping apparatus 15 in the apparatus 10 for production of a compound stretchable member 5. In the drawing illustrating production of the compound stretchable member 5, for the directions of the compound stretchable member 5 there are used a first direction D1 which is the same direction as the material machine direction MD, a second direction D2 which is the direction orthogonal to the first direction D1 on the surface of the compound stretchable member 5, and the thickness direction DT of the compound stretchable member 5.

Referring to Fig. 7, the continuous gear roll 15B has a plurality of continuous teeth 27 separated in the circumferential direction, each of the continuous teeth being continuous in the widthwise direction. Also, the cut-tooth gear roll 15A has a plurality of discontinuous teeth 29 separated in the circumferential direction, each of the discontinuous teeth 29 being discontinuous in the widthwise direction by one or more cut-tooth portions 31, the cut-tooth portions 31 being aligned in the circumferential direction.

In this production example, the discontinuous teeth 29 include large-pitch discontinuous teeth 29L that are to shape the recesses 51 and protrusions 53 in the concavoconvex regions 41 located in the large pitch area ALP of the compound stretchable member 5. The discontinuous teeth 29 also include small-pitch discontinuous teeth 29S that are to shape the recesses 51 and protrusions 53 in the concavoconvex regions 41 located in the small pitch area ASP of the compound stretchable member 5.

Also, in this production example, the continuous teeth 27 include large-pitch continuous teeth 27L having the same cross-sectional shape as the large-pitch discontinuous teeth 29L, at a location in the widthwise direction engaging with the large-pitch discontinuous teeth 29L. Similarly, the continuous teeth 27 include small-pitch continuous teeth 27S having the same cross-sectional shape as the small-pitch discontinuous teeth 29S, at a location in the widthwise direction engaging with the small-pitch discontinuous teeth 29S.

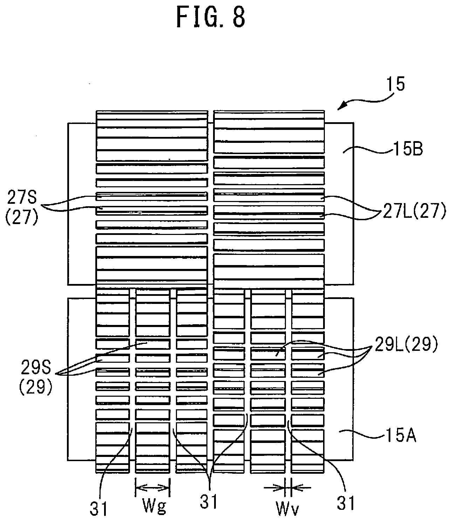

Fig. 8 is a front view of the cut-tooth gear roll 15A and continuous gear roll 15B of Fig. 7. For this production example, the width Wv of the cut-tooth portion 31 forming the non-concavoconvex regions 43 in the nonwoven fabric sheet 6B is 1 mm. The width Wg of the large-pitch discontinuous teeth 29L and small-pitch discontinuous teeth 29S is 4 mm. However, the dimensions of each constituent element of the cut-tooth gear roll 15A and continuous gear roll 15B are not limited to these dimensions.

For this production example, the large-pitch discontinuous teeth 29L and small-pitch discontinuous teeth 29S are each set in 3 rows in the second direction D2. However, the number of rows of discontinuous teeth 29 may be changed according to the size of the waistband portion WP and the dimensions of each location of the shaping apparatus 15.

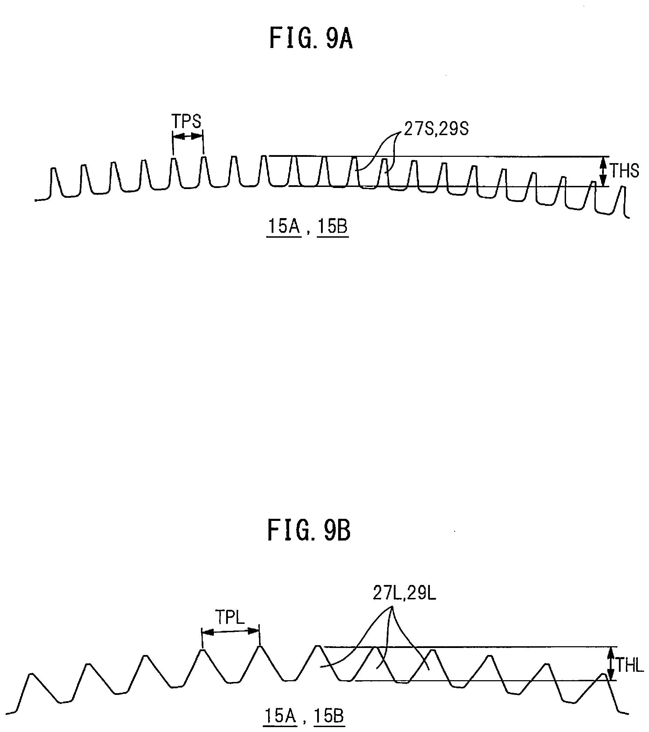

Fig. 9A is a partial end view of small-pitch discontinuous teeth 29S of the cut-tooth gear roll 15A and small-pitch continuous teeth 27S of the continuous gear roll 15B, of Fig. 7. Fig. 9B is a partial end view of large-pitch discontinuous teeth 29L of the cut-tooth gear roll 15A and large-pitch continuous teeth 27L of the continuous gear roll 15B, of Fig. 7. Since the small-pitch continuous teeth 27S and small-pitch discontinuous teeth have the same shapes as viewed from the side, and the large-pitch continuous teeth 27L and large-pitch discontinuous teeth 29L also have the same shapes as viewed from the side, their partial end views are shown in a single diagram. In this example, the heights THS of the small-pitch continuous teeth 27S and small-pitch discontinuous teeth 29S are approximately 1 mm, and the spacing TPS between the top sections of adjacent teeth 27S, 29S is approximately 1 mm. Also, the heights THL of the large-pitch continuous teeth 27L and large-pitch discontinuous teeth 29L are approximately 1 mm, and the spacings TPL between the top sections of adjacent teeth 27L, 29L are approximately 2 mm. Furthermore, while not shown, for this example the cut-tooth gear roll 15A and continuous gear roll 15B are mutually disposed so that the engagement length between the continuous teeth 27 and discontinuous teeth 29 is 0.8 mm. However, the dimensions of the teeth 27S, 29S, 27L, 29L of the cut-tooth gear roll 15A and continuous gear roll 15B and the placement of the cut-tooth gear roll 15A and continuous gear roll 15B are not limited to the manner described above.

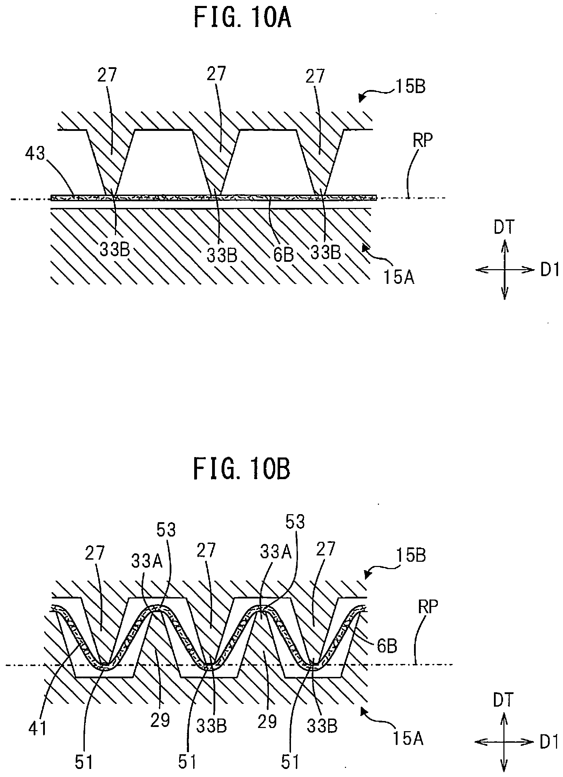

The preheated nonwoven fabric sheet 6B passes between the cut-tooth gear roll 15A and continuous gear roll 15B that are engaged and rotating in mutually opposite directions, as illustrated in Figs. 7 to 9B. The mechanism of shaping when the nonwoven fabric sheet 6B passes between the cut-tooth gear roll 15A and the continuous gear roll 15B, will now be explained with reference to Fig. 10A and Fig. 10B. Fig. 10A and Fig. 10B are magnified cross-sectional views schematically showing the area surrounding the meshing section between the cut-tooth gear roll 15A and continuous gear roll 15B, and a nonwoven fabric sheet 6 deformed between them, the view being linearly expanded in the peripheral direction of the cut-tooth gear roll 15A and continuous gear roll 15B. Fig. 10A is a cross-sectional view of a cut-tooth portion 31 of the cut-tooth gear roll 15A, and Fig. 10B is a cross-sectional view of a discontinuous tooth 29 portion of the cut-tooth gear roll 15A.

As shown in Fig. 10A, the nonwoven fabric sheet 6B that is introduced into the cut-tooth portion 31 of the cut-tooth gear roll 15A is pressed by the continuous gear roll 15B toward the outer side in the radial direction of the continuous gear roll 15B, but without being deformed, thus forming non-concavoconvex regions 43. Meanwhile, as shown in Fig. 10B, the nonwoven fabric sheet 6B that has been caught between the discontinuous teeth 29 of the cut-tooth gear roll 15A and the continuous teeth 27 of the continuous gear roll 15B becomes positioned at the tooth tip portion 33B. This causes stretching between the tooth tip portion 33B, the tooth tip portion 33A and another tooth tip portion 33B, which are adjacent in a three-point bending manner, forming a protrusion 53 with the tooth tip portion 33A as the top section.

Furthermore, stretching occurs between the tooth tip portion 33A, the tooth tip portion 33B and another tooth tip portion 33A, which are adjacent in a three-point bending manner, forming a recess 51 with the tooth tip portion 33B as the bottom section. During this time, the location in the radial direction of the gear rolls 15A, 15B is essentially the same for the non-concavoconvex regions 43 of the nonwoven fabric sheet 6 pressed to the continuous gear roll 15B at the cut-tooth portions 31 of the cut-tooth gear roll 15A, and the bottom sections of the recesses 51 of the nonwoven fabric sheet 6B which are positioned at the tooth tip portions 33B of the continuous gear roll 15B at the discontinuous tooth 29 portions of the cut-tooth gear roll 15A. That is, their location in the thickness direction DT of the nonwoven fabric sheet 6B are essentially the same. Thus, even after the concavoconvex regions 41 and the non-concavoconvex regions 43 have been formed in the nonwoven fabric sheet 6B, the non-concavoconvex regions 43 and the bottom sections of the recesses 51 of the nonwoven fabric sheet 6B are situated on essentially the same plane. This plane is defined as the imaginary reference plane RP for the nonwoven fabric sheet portions 6U, 6L. The reference plane RP is flat, and the non-concavoconvex regions 43 extend out on this reference plane RP. Since both of the nonwoven fabric sheet portions 6U, 6L are flexible, the reference plane RP is not necessarily flat.

Consequently, in terms of the relationship between the protrusions 53 and the reference plane RP, the protrusions 53 protrude from the reference plane RP in the thickness direction DT.

Furthermore, referring again to Fig. 6, the nonwoven fabric sheet 6B that has passed through the shaping apparatus 15 is conveyed to the merging zone 21 together with an upper nonwoven fabric sheet 6U formed in a step similar to the previous step, as the lower nonwoven fabric sheet 6L of the compound stretchable member 5.

Meanwhile, the elastic members 7 are kept wound on an elastic member feeder 17, and are wound out to be conveyed to the adhesive applicator 19. The elastic members 7 are subjected to a constant tension beforehand so as to be joined to the upper nonwoven fabric sheet 6U and the lower nonwoven fabric sheet 6L with the prescribed elastic member attachment factor (= (length of elastic member in extended state when attached to nonwoven fabric sheet) ÷ (length of elastic member in contracted state)). The following steps are carried out while maintaining the tension applied to the elastic members 7.

The adhesive applicator 19 applies an adhesive onto the elastic members 7 A that have been conveyed from the elastic member feeder 17.

In this production example, slit continuous coating is carried out at the adhesive applicator 19, and the elastic members 7A are matched to the sections where the adhesive has been discharged from the slit nozzles (not shown), for application of the adhesive around the elastic members 7A. The adhesive in this case is a hot-melt adhesive, but this is not limitative.

Moreover, at the merging zone 21, the lower nonwoven fabric sheet 6L is overlaid with an upper nonwoven fabric sheet 6U shaped by the same shaping apparatus as the shaping apparatus 15 that has shaped the lower nonwoven fabric sheet 6L as described above. Here, the upper nonwoven fabric sheet 6U and lower nonwoven fabric sheet 6L are overlaid so that the respective concavoconvex regions 41 and non-concavoconvex regions 43 are mutually aligned, and so that the protrusions 53U, 53L of the concavoconvex regions 41 are facing each other. As a result, the protrusions 53U, 53L of the nonwoven fabric sheets 6U, 6L enter into the recesses 51L, 51U of the nonwoven fabric sheets 6L, 6U. When the nonwoven fabric sheets 6U, 6L are to be overlaid, the adhesive-applied elastic members 7B are positioned between the non-concavoconvex regions 43 formed on the nonwoven fabric sheets 6U, 6L.

Finally, the upper nonwoven fabric sheet 6U, lower nonwoven fabric sheet 6L and elastic members 7B that have passed through the merging zone 21 are conveyed to the attachment press 25, and pressure is applied to the upper nonwoven fabric sheet 6U and lower nonwoven fabric sheet 6L in the thickness direction DT. This causes the nonwoven fabric sheets 6U, 6L to become joined together via the elastic members 7 in the non-concavoconvex regions 43, completing the final compound stretchable member 5.

In this production example, the nonwoven fabric sheets 6U, 6L are joined together via the elastic members 7 in the non-concavoconvex regions 43. As another example, however, the nonwoven fabric sheet portions 6U, 6L are joined at least partially via the elastic members 7 in the concavoconvex regions 41. As yet another example, the nonwoven fabric sheet portions 6U, 6L are joined at least partially in a manner not via the elastic members 7.

Incidentally, as explained at the beginning of the description of this embodiment, the disposable diaper 1 of this embodiment has the compound stretchable member 5 produced using the method described above provided in the waistband portion WP. According to this embodiment, the compound stretchable member 5 is disposed in the disposable diaper 1 in such a manner that the first direction D1 of the compound stretchable member 5 is essentially oriented in the same direction as the waist direction DW. In other words, the compound stretchable member 5 is disposed in the disposable diaper 1 so that the second direction D2 of the compound stretchable member 5 matches the direction orthogonal to the waist direction DW. Thus, the upper nonwoven fabric sheet 6U and lower nonwoven fabric sheet 6L form an inner side nonwoven fabric sheet portion 6U and outer side nonwoven fabric sheet portion 6L in the compound stretchable member 5.

Incidentally, although two nonwoven fabric sheets 6U, 6L were used in this production example, the inner side nonwoven fabric sheet portion 6U and outer side nonwoven fabric sheet portion 6L of the disposable diaper 1 may instead be constructed by folding the single nonwoven fabric sheet along folding lines parallel to the first direction D1.

This explains the function and effect of the disposable diaper 1 of this embodiment.

As mentioned above, the disposable diaper 1 of this embodiment includes concavoconvex regions 41 with mutually different concavoconvexity pitches. Specifically, the disposable diaper 1 includes a small pitch area ASP wherein the concavoconvexity pitch of the recesses 51 and protrusions 53 shaped in the concavoconvex regions 41 is small and a large pitch area ALP wherein the concavoconvexity pitch is large.

Since the concavoconvexity pitch is large in the large pitch area ALP, larger wrinkles are formed in the concavoconvex regions 41 than in the small pitch area ASP, when the compound stretchable member 5 undergoes contraction. This causes the concavoconvex regions 41L of the large pitch area ALP to thicken during contraction of the compound stretchable member 5. As a result, this increases the grip feel when the large pitch area ALP is gripped in order to lift the disposable diaper 1, and thus makes it easier for the disposable diaper 1 to be lifted.

On the other hand, since the concavoconvexity pitch is smaller in the small pitch area ASP, smaller wrinkles are formed than in the large pitch area ALP during contraction of the compound stretchable member 5, while the number of wrinkles is also increased, and the contact area with the body of the wearer increases. This improves the fitting property of the waistband portion WP in the small pitch area ASP, and helps to minimize leakage.

As explained above, in the disposable diaper 1 of this embodiment there are formed a small pitch area ASP that improves the fitting property and a large pitch area ALP that facilitates gripping and lifting. As a result, it is possible to provide a disposable diaper which has an improved fitting property at the waistband portion WP of the disposable diaper 1, while also being easily lifted when worn.

For this embodiment, the large pitch area ALP is provided further toward the waistband opening WO side than the small pitch area ASP, and the small pitch area ASP is provided further toward the leg opening LO side than the large pitch area ALP. Thus, the section near the waistband opening WO of the waistband portion WP is easier to grip, being the section that is normally gripped when the disposable diaper 1 is lifted. Furthermore, large wrinkles are formed in the large pitch area ALP, in contrast to the small pitch area ASP. This can increase the air permeability due to larger gaps with the skin surface of the wearer when worn, allowing sweat on the skin surface of the wearer to escape more easily and helping to minimize mustiness of the disposable diaper 1. Moreover, since the compressed stiffness of the concavoconvex regions 41 is lower in the large pitch area ALP than in the small pitch area ASP, it is possible to alleviate constriction by the waistband portion WP around the waist of the wearer, for comfortable wearing of the disposable diaper 1. On the other hand, the small pitch area ASP is disposed on the leg opening LO side of the waistband portion WP, or in other words, at a location near the section where body fluids are excreted, as the location of the disposable diaper 1 that requires an improved fitting property and leakage prevention. As a result, it is possible to provide a disposable diaper 1 exhibiting the function and effect of the large pitch area ALP described above, while also efficiently exhibiting the effect of the small pitch area ASP, without reducing the overall concavoconvexity pitch in the waistband portion WP.

Furthermore, as mentioned above, the number of wrinkles during contraction of the compound stretchable member 5 increases in the small pitch area ASP. Consequently, when the compound stretchable member 5 contracts, in the concavoconvex regions 41S located in the small pitch area ASP there are more nonwoven fabric portions located between the recesses 51 and protrusions 53 facing the thickness direction DT of the compound stretchable member 5, compared to the large pitch area ALP, and the heights of each of the wrinkles are lower. This allows the compressive force in the thickness direction DT to be supported more in the concavoconvex regions 41S of the small pitch area ASP, compared to the concavoconvex regions 41L of the large pitch area ALP, thereby increasing the compressed stiffness in the thickness direction DT. With this embodiment, therefore, providing the small pitch area ASP in a location overlapping with the location where the absorbing element 3 is present allows the absorbing element 3 to be supported by high compressed stiffness, and allows the absorbing element 3 to be closely fitted with the skin surface of the wearer. As a result, it is possible to efficiently minimize leakage of the disposable diaper 1.

For a similar purpose, although not particularly specified in the embodiment described above, the spacing between the elastic members 7 of the compound stretchable member 5 provided in the small pitch area ASP is preferably smaller than the spacing between the elastic members 7 of the compound stretchable member 5 provided in the large pitch area ALP, in the direction orthogonal to the waist direction DW. Furthermore, the elastic member attachment factor of the elastic members 7 of the compound stretchable member 5 provided in the small pitch area ASP is preferably greater than the elastic member attachment factor of the elastic members 7 of the compound stretchable member 5 provided in the region of the large pitch area ALS. This is because, in this embodiment, the small pitch area ASP is provided at a location on the leg opening LO side of the waistband portion WP, i.e. near the location where body fluids are excreted, making it possible to improve the fitting property of the disposable diaper 1, and thus minimize leakage, at the small pitch area ASP.

The dimensions of the large pitch area ALP and small pitch area ASP in the direction orthogonal to the waist direction DW, which are included in the compound stretchable member 5, may be determined as desired in consideration of the aforementioned function and effect. The required dimensions will differ depending on the size of the disposable diaper 1 and the wearer (infant, child, adult, etc.), but the proper dimensions are preferably determined by experimentation, for example, so that the aforementioned function and effect are appropriately exhibited.

Moreover, while not particularly specified for the embodiment described above, in the disposable diaper 1, preferably the density of the cross-section curve element in the waist direction DW of the concavoconvex regions 41L is no greater than 25/20 mm at the compound stretchable member 5 provided in the large pitch area ALP, and the density of the cross-section curve element in the waist direction DW of the concavoconvex regions 41S is 26/20 mm or greater at the compound stretchable member 5 provided in the small pitch area ASP, with the waistband portion WP at 50% extension in the waist direction DW. This is because if the density of the cross-section curve element is in this numerical range, it will be possible to further improve the fitting property in the small pitch area ASP, in the waist portion WP as described above, and it will be easier to grip at the large pitch area ALP, and thus to lift the disposable diaper 1. Incidentally, the density of the cross-section curve element is a numerical value proportional to the number of wrinkles formed in the concavoconvex regions 41, per unit length.

The state of 50% extension referred to above is the state in which the compound stretchable member 5 has stretched in the stretching direction to an elongation percentage of 50% (in the waist direction DW, for the embodiment described above). The elongation percentage is defined by the following formula.

$$\text{Elongation percentage (\%)} = (LM - LM0)/LM0 \cdot 100$$

Here, LM: Length of stretched compound stretchable member portion in the stretching direction

LM0: Length of compound stretchable member portion in the natural (contracted) state, in the stretching direction

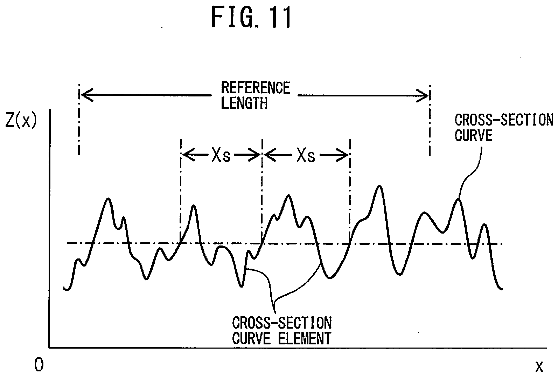

The cross-section curve element density is determined in the following manner. First, a cross-section curve along the stretching direction of the concavoconvex regions 41 in the compound stretchable member 5 is measured using a profile meter. The cross-sectional shape is measured at approximately the center between two mutually adjacent elastic members. Next, based on the cross-section curve, the height $Z(x)$ and length Xs of the cross-section curve element at a reference length is determined (see JIS B0601:2001(ISO4287:1997), JIS B0651:2001 (ISO3274:1996) and Fig. 11). Finally, the cross-section curve element density D is calculated from the average value PSm of the lengths Xs of the cross-section curve element (D = 1/PSm).

Examples

The tests carried out to confirm the function of the compound stretchable member will now be described. The testing was conducted for concavoconvex region thickness, concavoconvex region cross-section curve element density, flat air permeability and compression property, for a compound stretchable member including a large pitch area and a small pitch area.

The working example is a disposable diaper including region A located on the waistband opening side, corresponding to the large pitch area, and region B located on the leg opening side, corresponding to the small pitch area. The tooth pitch of the shaping apparatus used to shape the concavoconvex regions in region A (corresponding to the distances TPS, TPL between the top sections of teeth 27, 29 adjacent in the circumferential direction of the gear rolls 15A, 15B) was 2.0 mm, and the tooth pitch of the shaping apparatus used to shape the concavoconvex regions in region B was 1.0 mm. The comparative example is a disposable diaper including region C provided with a compound stretchable member without shaping of recesses and protrusions, located on the waistband portion side, and region D including concavoconvex regions shaped by a shaping apparatus having a tooth pitch of 1.5 mm, located on the leg opening sides. The construction of the other stretchable members in the example and comparative example will be understood by referring to Table 1 below.

The test methods for this test will now be described. For each test, a separate sample or test strip of the compound stretchable member located in each region A to D was prepared and each test was conducted separately. Also, except for measurement of the cross-section curve element density, the sample used was the compound stretchable member in the natural (contracted) state.

(Thickness)

The thickness of the compound stretchable member was measured using a thickness gauge FS-60DS by Daiei Kagaku Seiki Mfg. Co., Ltd., with a load of about 294.2 Pa (3 gf/cm$^2$) applied by a pressure plate having an area of 20 cm$^2$ (circular). The measurement was conducted 10 times in the concavoconvex region of the sample of the compound stretchable member. The arithmetic mean of the measured data was calculated to obtain the thickness of the compound stretchable member.

(Density of cross-section curve element)

The cross-section curve was measured using a profile measurement system KS-1100, laser sensor LK-G30 (spot diameter: 30 $\mu$m) and controller LK-GD500, by Keyence Corp. The measurement range was 0 to 30,000 $\mu$m, the measurement pitch was 5 $\mu$m, the measurement point number was 6001, the spot movement direction was the stretching direction, and the spot traveling speed was 50 $\mu$m/sec. A test strip 66, described below, was prepared from the sample 64 (120 mm width, 200 mm length), and the test strip 66 was measured. The moving average of ±12 points was conducted once to obtain cross-section curve data. The density of the cross-section curve element was calculated as described above, from the cross-section curve data.

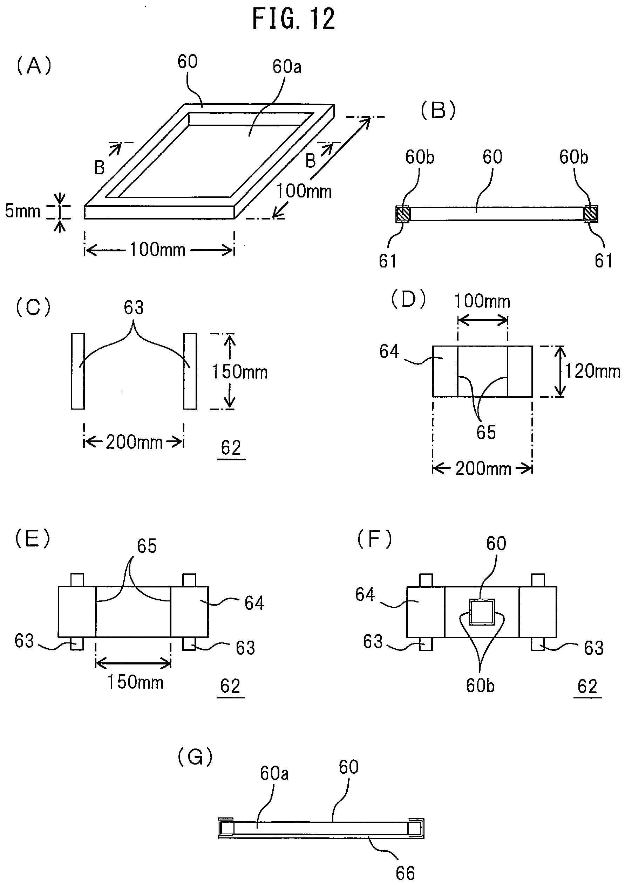

The test strip 66 for measurement of the cross-section curve was prepared in the following manner. Specifically, as shown in Fig. 12(A), a quadrilateral fixture 60 with an opening 60a was prepared. As shown in Fig. 12(B) which is a cross-sectional view along line B-B of Fig. 12(A), hook tape 61 was attached at each of two mutually facing sides 60b of the fixture 60. Also, as shown in Fig. 12(C), a pair of hook tapes 63 were attached to a table 62 at a spacing of 200 mm. Also, as shown in Fig. 12(D), a sample 64 (120 mm width, 200 mm length) of the compound stretchable member was prepared, and marks 65 were made at 100 mm spacings in the stretching direction (the direction in which the elastic members 7 extends) in the sample 64 in the natural (contracted) state.

Next, as shown in Fig. 12(E), the sample 64 was stretched evenly in the stretching direction until the spacing between the marks 65 reached 150 mm, and the hook tapes 63 were attached to the table 62. Then, as shown in Fig. 12(F), the fixture 60 was placed on the sample 64 so that the two sides 60b were orthogonal to the stretching direction of the sample 64, and the sample 64 was attached to the hook tape 61. The sample 64 was then peeled from the hook tape 63 on the table 62, and the sample 64 was attached to the hook tape 61 so as to be wrapped around the two sides 60b of the fixture 60. Next, the section of the sample 64 running over the fixture 60 was removed. The test strip 66 shown in Fig. 12(G) was fabricated in this manner. Measurement of the cross-section curve was carried out at the section located on the opening 60a of the fixture 60.

(Flat air permeability)

The flat air permeability was measured with an air permeability tester KES-F8-AP1 by Kato Tech Corp., using a sample with a size of 40 mm × 40 mm. The units for the flat air permeability were converted to [$m^3/m^2$/min]. The test strip was set in a tester, an acrylic board with a size of 100 mm × 100 mm was further set over it, and the measurement was conducted under a load of 3.5 mN/cm$^2$. The flat air permeability referred to here is the flat air permeability in the second direction of the compound stretchable member, or in other words, in the direction orthogonal to the waist direction of the disposable diaper. A larger numerical value for the flat air permeability signifies higher air permeability and thus greater ability to reduce mustiness of the disposable diaper.

(Compression property)

The WC value indicating the compression property was measured using an automated compression tester KES-FB3-AUTO-A by Kato Tech Corp. For the compression distance, the pressure plate area was 2 cm$^2$ (circular), the compression speed was 0.02 mm/sec, the stroke was 5 mm/10 V, the amp setting was SENS2, the uptake spacing was 0.1 second and the upper load limit was approximately 4.9 kPa (50 gf/cm$^2$). One measurement was made for each sample. A larger value for WC indicates easier compression. In other words, a smaller WC value indicates higher compressed stiffness.

Table 1 shows the compound stretchable member structures and test results for the example and comparative example. In Table 1, the "tooth engagement length" is the length of engagement between the teeth on the facing sides of the continuous gear roll and cut-tooth gear roll. Also, the "number used for elastic members" is the number of elastic yarns used per elastic member. The "elastic member spacing" is the spacing between elastic members of the compound stretchable member in the direction orthogonal to the waist direction.

[Table 1]

| | | Example | | Comparative Example | |
|---|---|---|---|---|---|
| Region name | | A region | B region | C region | D region |
| Compound stretchable member: | | | | | |
| Outer side nonwoven fabric type | | Spunbond nonwoven fabric | Spunbond nonwoven fabric | Spunbond nonwoven fabric | Spunbond nonwoven fabric |
| Outer side nonwoven fabric basis weight | g/m$^2$ | 17 | 17 | 17 | 17 |

(continued)

| | | Example | | Comparative Example | |
|---|---|---|---|---|---|
| Region name | | A region | B region | C region | D region |
| Compound stretchable member: | | | | | |
| Outer side nonwoven fabric type | | Spunbond nonwoven fabric | Spunbond nonwoven fabric | Spunbond nonwoven fabric | Spunbond nonwoven fabric |
| Inner side nonwoven fabric type | | SMS nonwoven fabric | SMS nonwoven fabric | SMS nonwoven fabric | SMS nonwoven fabric |
| Inner side nonwoven fabric basis weight | g/m$^2$ | 15 | 15 | 15 | 15 |
| Tooth pitch | Mm | 2.0 | 1.0 | --- | 1.5 |
| Tooth enqaqement length | Mm | 0.8 | 0.8 | --- | 1.2 |
| Elastic member material | | Urethane spandex | Urethane spandex | Urethane spandex | Urethane spandex |
| Elastic member fiber size | Dtex | 780 | 470 | 780 | 470 |
| Number used for elastic members | no. | 5 | 10 | 5 | 10 |
| Elastic member spacing | Mm | 9 | 5 | 9 | 5 |
| Elastic member attachment factor | Times | 2.4 | 2.8 | 2.4 | 2.8 |
| Test results: | | | | | |
| Thickness | Mm | 1.74 | 1.20 | 2.90 | 1.62 |
| Cross-section curve element density | no/20 mm | 20 | 34 | 7 | 28 |
| Flat air permeability | m$^3$/m$^2$/min | 24.11 | 10.81 | 9.83 | 22.58 |
| WC value (compression property) | N·m/m$^2$ | 0.80 | 0.57 | 2.19 | 0.99 |

Referring to Table 1, in the example, the A region corresponding to the large pitch area had a thicker concavoconvex region and a higher flat air permeability, than the B region corresponding to the small pitch area. Also, in the comparative example, the C region which was not shaped easily formed large wrinkles and therefore was thicker, but the flat air permeability was still low. Furthermore, in the comparative example, the D region was thinner and had lower flat air permeability than the A region of the example. Thus, it may be said that the compound stretchable member located in the A region of the example is easy to grip and has high air permeability.

The B region, which corresponded to the small pitch area, had higher cross-section curve element density and a lower WC value than the A region which corresponded to the large pitch area. Moreover, in the comparative example, the C region and D region had lower cross-section curve element density and a higher WC value than the B region of the example. Thus, the compound stretchable member located in the B region corresponding to the small pitch area may be said to have a greater area of contact with the body of the wearer and high compressed stiffness, and therefore an excellent fitting property.

This demonstrates that the disposable diaper of the example can exhibit an improved fitting property and can thus minimize leakage, and is easy to grip when lifted and therefore easy to lift.

All features that will be readily appreciated by a person skilled in the art from the present specification, the drawings and the description in the claims, though explained only in combinations related with certain other features throughout the present specification, may be employed either independently, or one or more of the disclosed features may be combined in any desired combination, so long as the features are not distinctly excluded and so long as the technical mode is not impossible or the combination is not meaningless.

For example, in the embodiment described above, the small pitch area ASP and large pitch area ALP are provided continuously in the direction orthogonal to the waist direction DW. According to a different embodiment, the small pitch area ASP and large pitch area ALP are provided separately in the direction orthogonal to the waist direction DW.

Also for this embodiment, the large pitch area ALP is provided on the waistband opening WO side, and the small pitch area ASP is provided further toward the leg opening LO side than the large pitch area ALP. For a different embodiment, the small pitch area ASP is provided on the waistband opening WO side, and the large pitch area ALP is provided further toward the leg opening LO side than the small pitch area ASP. This makes it easier to grip and lift the section near the leg opening LO of the waistband portion WP when the disposable diaper 1 is fitted onto the wearer.

Furthermore, in the compound stretchable member 5 of the disposable diaper 1 of the embodiment described above, the small pitch area ASP is provided at a location that overlaps the location where the absorbing element 3 is present, and the large pitch area ALP is provided at a location that does not overlap the location where the absorbing element 3 is present. In a separate embodiment, the large pitch area ALP is also provided in a location overlapping with the location in which the absorbing element 3 is present. Moreover, in yet a different embodiment, the large pitch area ALP is provided at a location that overlaps the location where the absorbing element 3 is present, and the small pitch area ASP is provided at a location that does not overlap the location where the absorbing element 3 is present.

In the disposable diaper 1 of the embodiment described above, the large pitch area ALP and small pitch area ASP of the compound stretchable member 5 are provided across the entire circumference in the waist direction DW of the waistband portion WP. However, either or both the large pitch area ALP and small pitch area ASP of the compound stretchable member 5 may be provided continuously or intermittently not on the entire circumference but on a part in the waist direction DW of the waistband portion WP. In addition, in a different embodiment, there is provided a separate region in which a concavoconvex region is formed provided with recesses 51 and protrusions 53 at a concavoconvexity pitch different from that of the concavoconvex regions 41L, 41S provided in the large pitch area ALP and small pitch area ASP. As a result, it is possible to arbitrarily provide an easily grippable region, or a region with an improved fitting property, not only in the direction orthogonal to the waist direction DW but even in the waist direction DW.

Furthermore, in the compound stretchable member 5 of the disposable diaper 1 of this embodiment, as shown in Fig. 5A, the inner side nonwoven fabric sheet portion 6U and outer side nonwoven fabric sheet portion 6L are overlaid in such a manner that the concavoconvex regions 41 of the inner side nonwoven fabric sheet portion 6U and the concavoconvex regions 41 of the outer side nonwoven fabric sheet portion 6L are mutually overlaid and aligned, and the non-concavoconvex regions 43 of the inner side nonwoven fabric sheet portion 6U and the non-concavoconvex regions 43 of the outer side nonwoven fabric sheet portion 6L are mutually overlaid and aligned. In a different embodiment, the concavoconvex regions 41 of the inner side nonwoven fabric sheet portion 6U and the concavoconvex regions 41 of the outer side nonwoven fabric sheet portion 6L are not aligned but instead are overlaid in a slightly shifted state in the direction orthogonal to the waist direction DW. That is, in this different embodiment, the non-concavoconvex regions 43 are overlaid with some of the concavoconvex regions 41 at the nonwoven fabric sheet portions 6U, 6L of the compound stretchable member 5.

Moreover, in the embodiment described above, the elastic members 7 are disposed between the non-concavoconvex regions 43 of the inner side nonwoven fabric sheet portion 6U and the non-concavoconvex regions 43 of the outer side nonwoven fabric sheet portion 6L. According to another embodiment, at least some of the elastic members 7 are disposed between the concavoconvex regions 41 of the inner side nonwoven fabric sheet portion 6U and the concavoconvex regions 41 of the outer side nonwoven fabric sheet portion 6L.

In the embodiment described above, the protrusions 53U, 53L of the nonwoven fabric sheets 6U, 6L enter into the recesses 51L, 51U of the nonwoven fabric sheets 6L, 6U. In yet a different embodiment, the protrusions 53U, 53L of the nonwoven fabric sheets 6U, 6L do not enter into the recesses 51L, 51U of the nonwoven fabric sheets 6L, 6U.

The present invention is prescribed as follows.

(1) A disposable diaper comprising a waistband portion, situated around the waist of the wearer and a crotch portion situated at the crotch of the wearer, and extending from the stomach side portion of the waistband portion to the back side portion of the waistband portion,
a compound stretchable member being provided in the waistband portion,
the compound stretchable member comprising a plurality of elastic members extending in the waist direction and an inner side nonwoven fabric sheet portion and outer side nonwoven fabric sheet portion that are overlaid so as to be mutually joined through the elastic members,

wherein the inner side nonwoven fabric sheet portion and the outer side nonwoven fabric sheet portion each comprise a plurality of concavoconvex regions at least partially comprising protrusions and recesses that alternately repeat along the waist direction and extend in the direction orthogonal to the waist direction, and

at least one non-concavoconvex region mutually separating the concavoconvex regions in the direction orthogonal to the waist direction,

the waistband portion including a first region, and a second region provided either continuously with or separate from the first region in the direction orthogonal to the waist direction,

the protrusions and recesses of the concavoconvex regions being provided at a first concavoconvexity pitch with the compound stretchable member disposed in the first region, and at a second concavoconvexity pitch that is different from the first concavoconvexity pitch, in the compound stretchable member disposed in the second region, along the waist direction.

(2) A disposable diaper according to (1), wherein

the first region is provided continuously with the second region in the direction orthogonal to the waist direction.

(3) A disposable diaper according to (1) or (2), wherein

the second region is provided further toward the crotch portion side than the first region, and

the first concavoconvexity pitch is longer than the second concavoconvexity pitch.

(4) A disposable diaper according to (3), wherein in the state in which the waistband portion is stretched 50% in the waist direction,

the density of the cross-section curve element in the waist direction of the concavoconvex regions is no greater than 25/20 mm at the compound stretchable member provided in the first region, and

the density of the cross-section curve element in the waist direction of the concavoconvex regions is 26/20 mm or greater at the compound stretchable member provided in the second region.

(5) A disposable diaper according to (3) or (4), wherein

an absorbing element is provided along the lengthwise direction of the crotch portion,

the second region is provided at a location overlapping with the location in which the absorbing element is present, and

the first region is provided at a location not overlapping with the location in which the absorbing element is present.

(6) A disposable diaper according to any one of (3) to (5), wherein

the spacing between the elastic members of the compound stretchable member provided in the second region is smaller than the spacing between the elastic members of the compound stretchable member provided in the first region.

(7) A disposable diaper according to any one of (3) to (6), wherein

the elastic member attachment factor of the elastic members of the compound stretchable member provided in the second region is greater than the elastic member attachment factor of the elastic members of the compound stretchable member provided in the first region.

(8) A disposable diaper according to any one of (1) to (7), wherein

the waistband portion includes the first region and second region in a portion of the waist direction, and

the waistband portion is provided with a separate region in which the concavoconvexity pitch of the protrusions and recesses provided in the concavoconvex regions differs from the first region and the second region.

(9) A disposable diaper according to any one of (1) to (8), wherein

the inner side nonwoven fabric sheet portion and the outer side nonwoven fabric sheet portion are overlaid in such a manner that the concavoconvex regions of the inner side nonwoven fabric sheet portion and the concavoconvex regions of the outer side nonwoven fabric sheet portion are mutually aligned, and that the non-concavoconvex regions of the inner side nonwoven fabric sheet portion and the non-concavoconvex regions of the outer side nonwoven fabric sheet portion are mutually aligned.

(10) A disposable diaper according to (9), wherein

the elastic members are disposed between the non-concavoconvex region of the inner side nonwoven fabric sheet portion and the non-concavoconvex region of the outer side nonwoven fabric sheet portion.

(11) A disposable diaper according to (9) or (10), wherein

the protrusions of the inner side nonwoven fabric sheet portion enter into the recesses of the outer side nonwoven fabric sheet portion, and the protrusions of the outer side nonwoven fabric sheet portion enter into the recesses of the inner side nonwoven fabric sheet portion.

(12) A method for producing a disposable diaper comprising a waistband portion, situated around the waist of the wearer and a crotch portion situated at the crotch of the wearer, and extending from the stomach side portion of the waistband portion to the back side portion of the waistband portion, the method for producing a disposable diaper including at least:

a step of producing a compound stretchable member comprising an inner side nonwoven fabric sheet portion and an outer side nonwoven fabric sheet portion which are mutually overlaid, and a plurality of elastic members extending in a first direction between the inner side nonwoven fabric sheet portion and the outer side nonwoven fabric sheet portion, and

a step of forming the disposable diaper using the compound stretchable member at the waistband portion, wherein the step of producing the compound stretchable member includes a shaping step, an overlaying step and a joining step,

in the shaping step, a plurality of concavoconvex regions at least partially comprising protrusions and recesses that alternately repeat along the first direction and extend in the second direction orthogonal to the first direction, and at least one non-concavoconvex region mutually separating the concavoconvex regions in the second direction, are formed in the inner side nonwoven fabric sheet portion and the outer side nonwoven fabric sheet portion, and the inner side nonwoven fabric sheet portion and the outer side nonwoven fabric sheet portion are shaped so that the compound stretchable member includes, in the concavoconvex regions, a first region with the protrusions and recesses provided at a first concavoconvexity pitch along the first direction, and in the concavoconvex region, a second region with the protrusions and recesses provided at a second concavoconvexity pitch which is different from the first concavoconvexity pitch, along the first direction, and so that the first region is continuous with or separate from the second region in the second direction,

in the overlaying step, the inner side nonwoven fabric sheet portion and the outer side nonwoven fabric sheet portion are overlaid,

in the joining step, the inner side nonwoven fabric sheet portion and the outer side nonwoven fabric sheet portion are joined together via the elastic members, and

in the step of forming the disposable diaper, the waistband portion is formed of the compound stretchable member by joining the compound stretchable member with the lengthwise ends of the absorbing element, so that the lengthwise direction of the absorbing element provided along the lengthwise direction of the crotch portion and the second direction of the compound stretchable member are flush.

Explanation of Symbols

1 Disposable diaper
3 Absorbing element
5 Compound stretchable member
6 Nonwoven fabric sheet
6L Outer side nonwoven fabric sheet portion (lower nonwoven fabric sheet)
6U Inner side nonwoven fabric sheet portion (upper nonwoven fabric sheet)
7 Elastic member
41 Concavoconvex region
43 Non-concavoconvex region
51 Recess
53 Protrusion
ALP Large pitch area (first region)
ASP Small pitch area (second region)
CP Crotch portion
DW Waist direction
WP Waistband portion
WPB Back side portion
WPS Stomach side portion

**Claims**

1. A disposable diaper (1) comprising a waistband portion (WP), situated around the waist of the wearer and a crotch portion (CP) situated at the crotch of the wearer, and extending from the stomach side portion of the waistband portion (WP) to the back side portion of the waistband portion,

a compound stretchable member (5) being provided in the waistband portion (WP),
the compound stretchable member (5) comprising a plurality of elastic members (7) extending in the waist direction and an inner side nonwoven fabric sheet portion (6U) and outer side nonwoven fabric sheet portion (6L) that are overlaid so as to be mutually joined through the elastic members (7),

wherein the inner side nonwoven fabric sheet portion (6U) and the outer side nonwoven fabric sheet portion (6L) each comprise

a plurality of concavoconvex regions (41) at least partially comprising protrusions (53) and recesses (51) that alternately repeat along the waist direction and extend in the direction orthogonal to the waist direction, and at least one non-concavoconvex region (43) mutually separating the concavoconvex regions in the direction orthogonal to the waist direction,

the waistband portion (WP) including a first region, and a second region provided either continuously with or separate from the first region in the direction orthogonal to the waist direction,

the protrusions (53) and recesses (51) of the concavoconvex regions (41) being provided at a first concavoconvexity pitch with the compound stretchable member (5) disposed in the first region, and at a second concavoconvexity pitch that is different from the first concavoconvexity pitch, in the compound stretchable member (5) disposed in the second region, along the waist direction.

2. A disposable diaper according to claim 1, wherein
the first region is provided continuously with the second region in the direction orthogonal to the waist direction.

3. A disposable diaper according to claim 1 or 2, wherein

the second region is provided further toward the crotch portion (CP) side than the first region, and
the first concavoconvexity pitch is longer than the second concavoconvexity pitch.

4. A disposable diaper according to claim 3, wherein in the state in which the waistband portion (WP) is stretched 50% in the waist direction,

the density of the cross-section curve element in the waist direction of the concavoconvex regions (41), as defined in the description, is no greater than 25/20 mm at the compound stretchable member (5) provided in the first region, and
the density of the cross-section curve element in the waist direction of the concavoconvex regions (41), as defined in the description, is 26/20 mm or greater at the compound stretchable member (5) provided in the second region.

5. A disposable diaper according to claim 3 or 4, wherein

an absorbing element (3) is provided along the lengthwise direction of the crotch portion (CP),
the second region is provided at a location overlapping with the location in which the absorbing element (3) is present, and
the first region is provided at a location not overlapping with the location in which the absorbing element (3) is present.

6. A disposable diaper according to any one of claims 3 to 5, wherein
the spacing between the elastic members (7) of the compound stretchable member (5) provided in the second region is smaller than the spacing between the elastic members of the compound stretchable member (5) provided in the first region.

7. A disposable diaper according to any one of claims 3 to 6, wherein
the elastic member attachment factor of the elastic members (7) of the compound stretchable member (5) provided in the second region is greater than the elastic member attachment factor of the elastic members (7) of the compound stretchable member (5) provided in the first region.

8. A disposable diaper according to any one of claims 1 to 7, wherein

the waistband portion (WP) includes the first region and second region in a portion of the waist direction, and
the waistband portion (WP) is provided with a separate region in which the concavoconvexity pitch of the protrusions (53) and recesses (51) provided in the concavoconvex regions (41) differs from the first region and the second region.

9. A disposable diaper according to any one of claims 1 to 8, wherein
the inner side nonwoven fabric sheet portion (6U) and the outer side nonwoven fabric sheet portion (6L) are overlaid in such a manner that the concavoconvex regions (41) of the inner side nonwoven fabric sheet portion and the concavoconvex regions of the outer side nonwoven fabric sheet portion are mutually aligned, and that the non-concavoconvex regions (43) of the inner side nonwoven fabric sheet portion (6U) and the non-concavoconvex regions (43) of the outer side nonwoven fabric sheet portion (6L) are mutually aligned.

10. A disposable diaper according to claim 9,
wherein the elastic members (7) are disposed between the non-concavoconvex region (43) of the inner side nonwoven fabric sheet portion (6U) and the non-concavoconvex region (43) of the outer side nonwoven fabric sheet portion (6L).

11. A disposable diaper according to claim 9 or 10, wherein
the protrusions (53) of the inner side nonwoven fabric sheet portion (6U) enter into the recesses (51) of the outer side nonwoven fabric sheet portion (6L), and the protrusions (53) of the outer side nonwoven fabric sheet portion enter into the recesses (51) of the inner side nonwoven fabric sheet portion.

12. A method for producing a disposable diaper (1) comprising a waistband portion (WP), situated around the waist of the wearer and a crotch portion (CP) situated at the crotch of the wearer, and extending from the stomach side portion of the waistband portion to the back side portion of the waistband portion (WP), the method for producing a disposable diaper (1) including at least:

a step of producing a compound stretchable member (5) comprising an inner side nonwoven fabric sheet portion (6U) and an outer side nonwoven fabric sheet portion (6L) which are mutually overlaid, and a plurality of elastic members (7) extending in a first direction between the inner side nonwoven fabric sheet portion and the outer side nonwoven fabric sheet portion, and
a step of forming the disposable diaper (1) using the compound stretchable member (5) at the waistband portion (WP),
wherein the step of producing the compound stretchable member (5) includes a shaping step, an overlaying step and a joining step,
in the shaping step, a plurality of concavoconvex regions (41) at least partially comprising protrusions (53) and recesses (51) that alternately repeat along the first direction and extend in the second direction orthogonal to the first direction, and at least one non-concavoconvex region (43) mutually separating the concavoconvex regions (41) in the second direction, are formed in the inner side nonwoven fabric sheet portion (6U) and the outer side nonwoven fabric sheet portion (6L), and the inner side nonwoven fabric sheet portion and the outer side nonwoven fabric sheet portion are shaped so that the compound stretchable member (5) includes, in the concavoconvex regions (41), a first region with the protrusions (53) and recesses (51) provided at a first concavoconvexity pitch along the first direction, and in the concavoconvex region, a second region with the protrusions (53) and recesses (51) provided at a second concavoconvexity pitch which is different from the first concavoconvexity pitch, along the first direction, and so that the first region is continuous with or separate from the second region in the second direction,
in the overlaying step, the inner side nonwoven fabric sheet portion (6U) and the outer side nonwoven fabric sheet portion (6L) are overlaid,
in the joining step, the inner side nonwoven fabric sheet portion (6U) and the outer side nonwoven fabric sheet portion (6L) are joined together via the elastic members (7), and
in the step of forming the disposable diaper (1), the waistband portion (WP) is formed of the compound stretchable member (5) by joining the compound stretchable member (5) with the lengthwise ends of an absorbing element (3), so that the lengthwise direction of the absorbing element (3) provided along the lengthwise direction of the crotch portion (CP) and the second direction of the compound stretchable member (5) are flush.

**Patentansprüche**

1. Eine Einwegwindel (1) umfassend einen Hüftbandteil (WP), der um die Taille des Trägers angeordnet ist, und einen Schrittteil (CP), der an dem Schritt des Trägers angeordnet ist und sich von dem Bauchseitenteil des Hüftbandteils (WP) zu dem Rückseitenteil des Hüftbandteils erstreckt,

ein dehnbares Verbundelement (5), das in dem Hüftbandteil (WP) vorgesehen ist,

EP 3 138 546 B1

das dehnbare Verbundelement (5), das eine Vielzahl von elastischen Elementen (7), die sich in der Taillenrichtung erstrecken, und einen Innenseitenfaservliesstofflagenteil (6U) und Außenseitenfaservliesstofflagenteil (6L), die überlagert sind, um durch die elastischen Elemente (7) gegenseitig verbunden zu sein, umfasst,
wobei der Innenseitenfaservliesstofflagenteil (6U) und Außenseitenfaservliesstofflagenteil (6L) jeweils
eine Vielzahl von konkav-konvexen Bereichen (41), die wenigstens teilweise Vorsprünge (53) und Aussparungen (51) umfassen, die sich abwechselnd entlang der Taillenrichtung wiederholen und sich in der Richtung orthogonal zu der Taillenrichtung erstrecken, und
wenigstens einen nicht-konkav-konvexen Bereich (43), der die konkav-konvexen Bereiche gegenseitig in der Richtung orthogonal zu der Taillenrichtung trennt, umfassen,
der Hüftbandteil (WP) einen ersten Bereich, und einen zweiten Bereich, der entweder kontinuierlich mit oder getrennt von dem ersten Bereich in der Richtung orthogonal zu der Taillenrichtung vorgesehen ist, umfasst,
die Vorsprünge (53) und Aussparungen (51) der konkav-konvexen Berieche (41), die an einer ersten Konkav-konvexitätsteilung mit dem dehnbaren Verbundelement (5) versehen werden, das in dem ersten Bereich angeordnet ist, und an einer zweiten Konkav-konvexitätsteilung, die sich von der ersten Konkav-konvexitätsteilung unterscheidet, in dem dehnbaren Verbundelement (5), das in dem zweiten Bereich angeordnet ist, entlang der Taillenrichtung.

2. Eine Einwegwindel gemäß Anspruch 1, wobei der erste Bereich kontinuierlich mit dem zweiten Bereich in der Richtung orthogonal zu der Taillenrichtung versehen ist.

3. Eine Einwegwindel gemäß Anspruch 1 oder 2, wobei

der zweite Bereich näher an dem Schrittteil (CP) als der erste Bereich vorgesehen ist, und
die erste Konkav-konvexitätsteilung länger als die zweite Konkav-konvexitätsteilung ist.

4. Eine Einwegwindel gemäß Anspruch 3, wobei in dem Zustand in dem der Hüftbandteil (WP) 50 % in der Taillenrichtung gedehnt ist,

die Dichte des Querschnittbogenelements in der Taillenrichtung der konkav-kovexen Bereiche (41), wie in der Beschreibung definiert, nicht mehr als 25/20 mm an dem dehnbaren Verbundelement (5), das in dem ersten Bereich vorgesehen ist, beträgt, und
die Dichte des Querschnittbogenelements in der Taillenrichtung der konkav-kovexen Bereiche (41), wie in der Beschreibung definiert, 26/20 mm oder mehr an dem dehnbaren Verbundelement (5), das in dem zweiten Bereich vorgesehen ist, beträgt.

5. Eine Einwegwindel gemäß Anspruch 3 oder 4, wobei

ein absorbierendes Element (3) entlang der Längsrichtung des Schrittteils (CP) vorgesehen ist,
der zweite Bereich an einer Stelle, die mit der Stelle, an der das absorbierende Element (3) vorliegt, überlappt, vorgesehen ist, und
der erste Bereich an einer Stelle, die mit der Stelle, an der das absorbierende Element (3) vorliegt, nicht überlappt, vorgesehen ist.

6. Eine Einwegwindel gemäß einem der Ansprüche 3 bis 5, wobei
der Abstand zwischen den elastischen Elementen (7) der dehnbaren Verbundelemente (5), die in dem zweiten Bereich vorgesehen sind, kleiner ist als der Abstand zwischen den elastischen Elementen der dehnbaren Verbundelemente (5), die in dem ersten Bereich vorgesehen sind.

7. Eine Einwegwindel gemäß einem der Ansprüche 3 bis 6, wobei
der Elastischeselementanlagerungsfaktor des elastischen Elements (7) des elastischen Verbundelements (5), das in dem zweiten Bereich vorgesehen ist, größer ist als der Elastischeselementanlagerungsfaktor des elastischen Elements (7) des elastischen Verbundelements (5), das in dem ersten Bereich vorgesehen ist.

8. Eine Einwegwindel gemäß einem der Ansprüche 1 bis 7, wobei

der Hüftbandteil (WP) den ersten Bereich und zweiten Bereich in einem Teil der Taillenrichtung umfasst, und
der Hüftbandteil (WP) mit einem separaten Bereich, in dem die Konkav-konvexitätsteilung der Vorsprünge (53) und Aussparungen (51), die in den konkav-konvexen Bereichen (41) vorgesehen sind, von dem ersten Bereich

18

und dem zweiten Bereich abweicht, versehen ist.

9. Eine Einwegwindel gemäß einem der Ansprüche 1 bis 8, wobei der Innenseitenfaservliesstofflagenteil (6U) und der Außenseitenfaservliesstofflagenteil (6L) auf so eine Weise überlagert sind, dass die konkav-konvexen Bereiche (41) des Innenseitenfaservliesstofflagenteils und die konkav-konvexen Bereiche des Außenseitenfaservliesstofflagenteils gegenseitig ausgerichtet sind.

10. Eine Einwegwindel gemäß Anspruch 9, wobei die elastischen Elemente (7) zwischen dem nicht-konkav-konvexen Bereich (43) des Innenseitenfaservliesstofflagenteil (6U) und dem nicht-konkav-konvexen Bereich (43) des Außenseitenfaservliesstofflagenteil (6L) angeordnet sind,

11. Eine Einwegwindel gemäß Anspruch 9 oder 10, wobei die Vorsprünge (53) des Innenseitenfaservliesstofflagenteils (6U) in die Aussparungen (51) des Außenseitenfaservliesstofflagenteils (6L) eintreten, und die Vorsprünge (53) des Außenseitenfaservliesstofflagenteils in die Aussparungen (51) des Innenseitenfaservliesstofflagenteils eintreten.

12. Ein Verfahren zur Herstellung einer Einwegwindel (1), umfassend einen Hüftbandteil (WP), der um die Taille des Trägers angeordnet ist, und einen Schrittteil (CP), der an dem Schritt des Trägers angeordnet ist und sich von dem Bauchseitenteil des Hüftbandteils zu dem Rückseitenteil des Hüftbandteils (WP) erstreckt, das Verfahren zur Herstellung einer Einwegwindel (1) wenigstens umfassend:

einen Schritt des Herstellens eines dehnbaren Verbundelements (5), das einen Innenseitenfaservliesstofflagenteil (6U) und einen Außenseitenfaservliesstofflagenteil (6L), die gegenseitig überlagert sind, umfasst, und eine Vielzahl von elastischen Elementen (7), die sich in einer ersten Richtung zwischen dem Innenseitenfaservliesstofflagenteil und dem Außenseitenfaservliesstofflagenteil erstrecken, und einen Schritt des Ausbildens der Einwegwindel (1) mittels des dehnbaren Verbundelements (5) an dem Hüftbandteil (WP),

wobei der Schritt des Herstellens des dehnbaren Verbundelements (5) einen Ausbildungsschritt, einen Überlappungsschritt und einen Verbindungsschritt umfasst, in dem Ausbildungsschritt, eine Vielzahl von konkav-konvexen Bereichen (41), die wenigstens teilweise Vorsprünge (53) und Aussparungen (51) umfassen, die sich abwechselnd entlang der ersten Richtung wiederholen und sich in der zweiten Richtung orthogonal zu der ersten Richtung erstrecken, und wenigstens ein nicht-konkav-konvexen Bereich (43), der die konkav-konvexen Bereiche (41) gegenseitig in der zweiten Richtung trennt, in dem Innenseitenfaservliesstofflagenteil (6U) und dem Außenseitenfaservliesstofflagenteil (6L) ausgebildet werden und der Innenseitenfaservliesstofflagenteil und der Außenseitenfaservliesstofflagenteil ausgebildet sind, sodass das dehnbare Verbundelement (5), in den konkav-konvexen Bereichen (41), einen ersten Bereich mit Vorsrpüngen (53) und Aussparungen (51), die an einer ersten Konkav-konvexitätsteilung entlang der ersten Richtung vorgesehen sind, und in dem konkav-konvexen Bereich, einen zweiten Bereich mit Vorsrpüngen (53) und Aussparungen (51), die an einer zweiten Konkav-konvexitätsteilung, die sich von der ersten Konkav-konvexitätsteilung unterscheidet, entlang der ersten Richtung vorgesehen sind, und so, dass der erste Bereich kontinuiertlich mit oder getrennt von dem zweiten Bereich in der zweiten Richtung ist, umfasst, in dem Überlappungsschritt, der Innenseitenfaservliesstofflagenteil (6U) und der Außenseitenfaservliesstofflagenteil (6L) überlagert werden, in dem Verbindungsschritt, der Innenseitenfaservliesstofflagenteil (6U) und der Außenseitenfaservliesstofflagenteil (6L) durch die elastischen Elemente (7) miteinander verbunden werden, und in dem Schritt des Ausbildens der Einwegwindel (1), der Hüftbandteil (WP) aus dem dehnbaren Verbundelement (5) durch Verbinden des denbahren Verbundteils (5) mit den Längsenden eines absorbierenden Elements (3), sodass die Längsrichtung des absorbierenden Elements (3), das entlang der Längsrichtung des Schrittteils (CP) und der zweiten Richtung des dehnbaren Verbundelements (5) bündig sind, ausgebildet ist.

**Revendications**

1. Couche jetable (1) comprenant une partie de ceinture (WP) située autour de la taille du porteur et une partie d'entrejambes (CP) située au niveau de l'entrejambes du porteur, et s'étendant de la partie côté ventre de la partie de ceinture (WP) à la partie côté dos de la partie de ceinture, un élément étirable composé (5) étant disposé dans la partie de ceinture (WP), l'élément étirable composé (5) comprenant une pluralité d'éléments élastiques (7) s'étendant dans la direction de taille et une partie de feuille de tissu non-tissé de côté interne (6U) et une partie de feuille de tissu non-tissé de côté externe (6L) qui sont recouvertes de manière à être mutuellement assemblées par le biais des éléments élastiques (7),

dans laquelle la partie de feuille de tissu non-tissé de côté interne (6U) et la partie de feuille de tissu non-tissé de côté externe (6L) comprennent chacune

une pluralité de régions concavo-convexes (41) comprenant au moins partiellement des saillies (53) et des évidements (51) qui se répètent en alternance le long de la direction de taille et s'étendent dans la direction orthogonale à la direction de taille, et au moins une région non concavo-convexe (43) séparant mutuellement les régions concavo-convexes dans la direction orthogonale à la direction de taille,

la partie de taille (WP) comprenant une première région, et une seconde région disposée soit en continu avec, soit séparée de la première région dans la direction orthogonale à la direction de taille,

les saillies (53) et évidements (51) des régions concavo-convexes (41) étant disposés à un premier pas de concavo-convexité avec l'élément étirable composé (5) disposé dans la première région, et à un second pas de concavo-convexité qui est différent du premier pas de concavo-convexité, dans l'élément étirable composé (5) disposé dans la seconde région, le long de la direction de taille.

**2.** Couche jetable selon la revendication 1, dans laquelle
la première région est disposée en continu avec la seconde région dans la direction orthogonale à la direction de taille.

**3.** Couche jetable selon la revendication 1 ou 2, dans laquelle
la seconde région est disposée davantage en direction du côté de la partie d'entrejambes (CP) que la première région, et
le premier pas de concavo-convexité est plus long que le second pas de concavo-convexité.

**4.** Couche jetable selon la revendication 3, dans laquelle dans l'état dans laquelle la partie de ceinture (WP) est étirée de 50 % dans la direction de taille,
la densité de l'élément de courbe de section transversale dans la direction de taille des régions concavo-convexes (41), telle que définie dans la description, n'est pas supérieure à 25/20 mm au niveau de l'élément étirable composé (5) disposé dans la première région, et
la densité de l'élément de courbe de section transversale dans la direction de taille des régions concavo-convexes (41), telle que définie dans la description, est égale ou supérieure à 26/20 mm au niveau de l'élément étirable composé (5) disposé dans la seconde région.

**5.** Couche jetable selon la revendication 3 ou 4, dans laquelle
un élément absorbant (3) est disposé le long de la direction longitudinale de la partie d'entrejambes (CP),
la seconde région est disposée au niveau d'un emplacement chevauchant l'emplacement dans lequel l'élément absorbant (3) est présent, et
la première région est disposée au niveau d'un emplacement ne chevauchant pas l'emplacement dans lequel l'élément absorbant (3) est présent.

**6.** Couche jetable selon l'une quelconque des revendications 3 à 5, dans laquelle
l'espacement entre les éléments élastiques (7) de l'élément étirable composé (5) disposé dans la seconde région est inférieur à l'espacement entre les éléments élastiques de l'élément étirable composé (5) disposé dans la première région.

**7.** Couche jetable selon l'une quelconque des revendications 3 à 6, dans laquelle
le facteur de fixation d'élément élastique des éléments élastiques (7) de l'élément étirable composé (5) disposé dans la seconde région est supérieur au facteur de fixation d'élément élastique des éléments élastiques (7) de l'élément étirable composé (5) disposé dans la première région.

**8.** Couche jetable selon l'une quelconque des revendications 1 à 7, dans laquelle
la partie de ceinture (WP) comprend la première région et la seconde région dans une partie de la direction de taille, et
la partie de ceinture (WP) est dotée d'une région séparée dans laquelle le pas de concavo-convexité des saillies (53) et évidements (51) disposés dans les régions concavo-convexes (41) diffère de celui des première région et seconde région.

**9.** Couche jetable selon l'une quelconque des revendications 1 à 8, dans laquelle
la partie de feuille de tissu non-tissé de côté interne (6U) et la partie de feuille de tissu non-tissé de côté externe (6L) sont recouvertes de sorte que les régions concavo-convexes (41) de la partie de feuille de tissu non-tissé de côté interne et les régions concavo-convexes de la partie de feuille de tissu non-tissé de côté externe sont mutuellement alignées, et que les régions non concavo-convexes (43) de la partie de feuille de tissu non-tissé de côté interne (6U) et les régions non concavo-convexes (43) de la partie de feuille de tissu non-tissé de côté externe (6L) sont mutuellement alignées.

10. Couche jetable selon la revendication 9,
dans laquelle les éléments élastiques (7) sont disposés entre la région non concavo-convexe (43) de la partie de feuille de tissu non-tissé de côté interne (6U) et la région non concavo-convexe (43) de la partie de feuille de tissu non-tissé de côté externe (6L).

11. Couche jetable selon la revendication 9 ou 10, dans laquelle
les saillies (53) de la partie de feuille de tissu non-tissé de côté interne (6U) pénètrent dans les évidements (51) de la partie de feuille de tissu non-tissé de côté externe (6L), et les saillies (53) de la partie de feuille de tissu non-tissé de côté externe pénètrent dans les évidements (51) de la partie de feuille de tissu non-tissé de côté interne.

12. Procédé de production d'une couche jetable (1) comprenant une partie de ceinture (WP), située autour de la taille du porteur et une partie d'entrejambes (CP) située au niveau de l'entrejambes du porteur, et s'étendant de la partie côté ventre de la partie de ceinture à la partie côté dos de la partie de ceinture (WP), le procédé de production d'une couche jetable (1) comprenant au moins :

une étape de production d'un élément étirable composé (5) comprenant une partie de feuille de tissu non-tissé de côté interne (6U) et une partie de feuille de tissu non-tissé de côté externe (6L) qui sont mutuellement recouvertes, et une pluralité d'éléments élastiques (7) s'étendant dans une première direction entre la partie de feuille de tissu non-tissé de côté interne et la partie de feuille de tissu non-tissé de côté externe, et une étape de formation de la couche jetable (1) au moyen de l'élément étirable composé (5) au niveau de la partie de ceinture (WP),
dans laquelle l'étape de production de l'élément étirable composé (5) comprend une étape de mise en forme, une étape de recouvrement et une étape d'assemblage,
dans l'étape de mise en forme, une pluralité de régions concavo-convexes (41) comprenant au moins partiellement des saillies (53) et des évidements (51) qui se répètent en alternance le long de la première direction et s'étendent dans la seconde direction orthogonale à la première direction, et au moins une région non concavo-convexe (43) séparant mutuellement les régions concavo-convexes (41) dans la seconde direction, est formée dans la partie de feuille de tissu non-tissé de côté interne (6U) et la partie de feuille de tissu non-tissé de côté externe (6L), et la partie de feuille de tissu non-tissé de côté interne et la partie de feuille de tissu non-tissé de côté externe sont mises en forme de sorte que l'élément étirable composé (5) comprend, dans les régions concavo-convexes (41), une première région avec les saillies (53) et évidements (51) disposés à un premier pas de concavo-convexité le long de la première direction, et dans la région concavo-convexe, une seconde région avec les saillies (53) et évidements (51) disposés à un second pas de concavo-convexité qui est différent du premier pas de concavo-convexité, le long de la première direction, et de sorte que la première région est continue avec, ou séparée de la seconde région dans la seconde direction,
dans l'étape de recouvrement, la partie de feuille de tissu non-tissé de côté interne (6U) et la partie de feuille de tissu non-tissé de côté externe (6L) sont recouvertes,
dans l'étape d'assemblage, la partie de feuille de tissu non-tissé de côté interne (6U) et la partie de feuille de tissu non-tissé de côté externe (6L) sont assemblées l'une à l'autre via les éléments élastiques (7), et
dans l'étape de formation de la couche jetable (1), la partie de ceinture (WP) est formée de l'élément étirable composé (5) en assemblant l'élément étirable composé (5) avec les extrémités longitudinales d'un élément absorbant (3), de sorte que la direction longitudinale de l'élément absorbant (3) disposé le long de la direction longitudinale de la partie d'entrejambes (CP) et la seconde direction de l'élément étirable composé (5) sont au même niveau.

# FIG. 1

# FIG. 2

WPB (WP)  CP  WPS (WP)

1

3

5  5

DW

EP 3 138 546 B1

# FIG. 3

FIG. 4A

FIG. 4B

# FIG. 5

FIG. 6

# FIG. 7

# FIG. 8

# FIG. 9A

# FIG. 9B

# FIG. 10A

# FIG. 10B

# FIG. 11

# FIG. 12

(A)

60    60a

5mm

100mm

100mm

(B)

60b    60    60b

61    61

(C)

63

150mm

200mm

62

(D)

100mm

64

120mm

65

200mm

(E)

65

64

63    63

150mm

62

(F)

60

64

63    63

60b

62

(G)

60a    60

66

**EP 3 138 546 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008173286 A
- US 2011251576 A1
- WO 2013054923 A1
- EP 2767267 A